# FASCICULE DE BREVET EUROPEEN

(11) **EP 2 878 589 B1**
(45) Date de publication et mention de la délivrance du brevet: **11.10.2017**
(21) Numéro de dépôt: 14306770.0
(22) Date de dépôt: 05.11.2014
(51) Int. Cl.: C07C 29/80, B01D 3/10, C07C 31/20

(54) **PROCÉDÉ FLEXIBLE POUR LE TRAITEMENT DE SOLVANT UTILISÉ DANS L'EXTRACTION DU GAZ NATUREL**
FLEXIBLES VERFAHREN FÜR DIE AUFBEREITUNG VON LÖSUNGSMITTELN, DAS ZUM ABBAU VON ERDGAS VERWENDET WIRD
FLEXIBLE METHOD FOR THE TREATMENT OF A SOLVENT USED IN THE EXTRACTION OF NATURAL GAS

(30) Priorité: 22.11.2013 FR 1361500
(43) Date de publication de la demande: 03.06.2015
(73) Titulaire: Prosernat, 92042 Paris la Defense (FR)
(72) Inventeur: Esquier, Jérémie, 92000 NANTERRE (FR); Chambon, Bernard, 78260 ACHERES (FR); Streicher, Christian, 92500 RUEIL-MALMAISON (FR)
(74) Mandataire: Schmitt, Nicolas A.J.

(56) Documents cités:
- EP-A1- 1 415 965
- WO-A1-98/39076
- WO-A1-2005/092470
- WO-A1-2010/080038
- GB-A- 2 467 169

## Description

L'invention concerne un procédé de purification d'un solvant, en particulier du monoéthylène glycol, qui est utilisé sur les champs de gaz naturel pour éviter la formation d'hydrates. Ledit solvant a un point d'ébullition supérieur à celui de l'eau, et est , à un moment au moins, en mélange avec de l'eau et des sels.

Ces hydrates se forment en présence d'eau, de gaz naturel et dans les conditions favorisant leur stabilité.

Leur présence peut devenir extrêmement nocive pour l'exploitation puisque des bouchons peuvent se former dans les tubes d'extraction ou dans les canalisations de transport, qui peuvent amener à un arrêt de la production.

Le problème se pose avec une acuité particulière pour les exploitations en plateformes en mer où le gaz est extrait dans un milieu relativement froid favorable à la formation d'hydrates et où par ailleurs les traitements du gaz sont reportés sur terre, et donc le gaz tel qu'extrait est envoyé vers l'installation sur terre et à des températures où les hydrates sont stables. Pour éviter ces inconvénients, il est connu d'utiliser des inhibiteurs de formation d'hydrates tels que le monoéthylène glycol (MEG).

Cette solution demeure onéreuse, vu les quantités nécessaires d'inhibiteur. Il fallait donc trouver un moyen pour recycler l'inhibiteur.

Il est connu, par exemple par les brevets de la société CCR Technologies, un procédé pour purifier le MEG et le recycler.

Ainsi le brevet EP-1261410 décrit une installation et un procédé de purification d'inhibiteur de formation d'hydrates tel que le MEG. Le MEG à traiter est envoyé dans un ballon de flash ou une colonne opérant sous vide de façon à séparer un flux liquide en fond de ballon (ou colonne) comprenant du MEG et les sels, et en tête de ballon (ou colonne) un flux essentiellement gazeux comprenant l'eau et du MEG. Le flux de MEG et sels est en partie réchauffé pour être recyclé au ballon (ou colonne), l'autre partie est purgée, la quantité purgée dépendant de la concentration en sels. Le flux de tête est distillé sous vide pour séparer l'eau et les gaz (en tête) et récupérer le MEG purifié (en fond) qui est recyclé sur le champ de gaz naturel.

Ce procédé fonctionne en mode dit de reclaiming.

Il est connu un autre mode dit de régénération pour purifier le MEG. Dans ce mode, le MEG à traiter est distillé sous pression atmosphérique pour séparer l'eau en tête de colonne, le flux de MEG et les sels sortant en fond de colonne est traité de façon à séparer les sels, par exemple au moyen d'un ballon ou colonne sous vide. Les demandes de brevet WO-2010/080038 et GB-2467169 décrivent des procédés pour purifier un solvant inhibiteur d'hydrates avec une phase de régénération et une phase de « reclaiming ».

Le déposant a constaté que la phase de reclaiming est coûteuse en énergie car il est nécessaire de faire circuler et réchauffer des quantités importantes de solvant (par ex MEG). Par ailleurs, le travail sur champ montre que le solvant (par ex MEG) peut à certains moments ne pas contenir d'eau de formation (eau chargée des sels de la formation traversée) tout comme à d'autres moments il peut en contenir de grandes quantités, cela dépend des formations traversées. Ce phénomène est aléatoire et jusqu'à ce jour difficile à prévoir.

Un objectif de l'invention est de réduire les coûts énergétiques du procédé tout en maintenant un bon niveau de purification.

Plus précisément, l'invention concerne un procédé de purification d'un solvant inhibiteur de la formation d'hydrates , ledit solvant ayant un point d'ébullition supérieur à celui de l'eau et étant , à un moment au moins, en mélange avec de l'eau et des sels , procédé qui comprend:
- éventuellement, un prétraitement dudit solvant à traiter, ledit prétraitement séparant au moins en partie les hydrocarbures, les condensats et les gaz, et ledit prétraitement comportant éventuellement d'addition d'agent chimique de neutralisation,
- une phase de reclaiming mise en oeuvre lorsque la teneur en sels dudit solvant à traiter, éventuellement prétraité, atteint le seuil de précipitation dans le mélange traité
- ladite phase comprenant un flash sous vide, opérant sous une pression inférieure à la pression atmosphérique et préférentiellement comprise entre 0.2 et 0.5 bar absolus et à une température inférieure à la température de dégradation du solvant , par lequel il est obtenu un flux de solvant contenant les sels et un flux vaporisé de solvant et d'eau, ledit flux de solvant et d'eau est distillé sous vide, à une pression sensiblement égale à celle régnant dans le dit flash sous vide pour séparer l'eau et récupérer un flux de solvant purifié, les sels sont séparés dudit flux de solvant contenant les sels , puis le solvant obtenu est recyclé au flash,
- lorsque ladite teneur en sels dans le mélange traité est inférieure au seuil de précipitation , la phase de reclaiming est arrêtée et une phase dite de régénération est mise en oeuvre,
- ladite phase de régénération comprenant la suppression du vide, le solvant à traiter, éventuellement prétraité, subissant une étape de flash , opérant sous une pression égale ou supérieure à la pression atmosphérique et préférentiellement comprise entre 1 et 2 bar absolus et à une température inférieure à la température de dégradation du solvant, par laquelle il est obtenu un premier flux de solvant purifié et un flux de solvant et d'eau, ledit flux de solvant et d'eau est distillé sous une pression sensiblement égale à celle régnant dans ledit flash , l'eau est séparée et un deuxième flux de solvant purifié est obtenu, ledit deuxième flux étant mélangé au dit premier flux, et/ou recyclé au dit flash.

Le procédé s'applique particulièrement bien au monoéthylène glycol.

Le solvant à traiter (en particulier le MEG) est inhibiteur de la formation d'hydrates lors du traitement de gaz.

Le solvant à traiter (en particulier le MEG) ne subit pas de traitement pour séparer les sels divalents . Les sels divalents restent présents dans le solvant entrant dans le flash.

Le niveau de purification du solvant (en particulier MEG) souhaité et obtenu est supérieur à 60 % pds et préférentiellement supérieur à 80 % pds.

L'eau issue du procédé contient généralement moins de 1 % pds de solvant (MEG en particulier) et préférentiellement moins de 0.1 % pds ; elle peut être réutilisée.

L'invention concerne également une installation de purification d'un solvant inhibiteur de la formation d'hydrates , ledit solvant ayant un point d'ébullition supérieur à celui de l'eau et étant , à un moment au moins, en mélange avec de l'eau et des sels, ladite installation comprenant:
- un ballon de flash 7 muni d'une canalisation 6 d'introduction du solvant à traiter, d'une canalisation 8 de sortie d'un mélange de solvant et d'eau, d'une canalisation 9 de sortie d'un mélange de solvant et de sels,
- un moyen 10 de séparation des sels muni d'une canalisation 9bis d'introduction du mélange de solvant et de sels, ladite canalisation 9bis comportant une vanne 16, le moyen 10 étant également muni d'une canalisation 11 de sortie des sels séparés et d'une canalisation 12 de sortie du solvant séparé des sels, ladite canalisation 12 étant reliée au ballon de flash 7 et munie d'une vanne 26,
- une colonne de distillation 13 pouvant opérer sous vide, munie d'une canalisation 6 d'introduction 8 dudit mélange de solvant et d'eau, d'une canalisation de sortie 15 du solvant purifié située en fond de colonne, d'une canalisation de sortie 14 de l'eau et de gaz, située en tête de colonne,
- une canalisation 17 munie d'une vanne 18 reliant ladite canalisation 9 à la canalisation 15 de sortie du solvant purifié
- un système de mise sous vide 20 pouvant délivrer une pression réduite dans la colonne de distillation et le ballon de flash, une canalisation 19 de by-pass dudit système et des vannes 24 et 25 au niveau respectivement dudit système et de la canalisation de by-pass,
- une canalisation 29 munie d'une vanne 28, reliant la canalisation 15 au ballon de flash 7,
et ladite installation fonctionnant selon 2 phases selon la teneur en sels du solvant délivrée par le moyen de dosage:
- dans l'une des phases, les vannes 18, 25 et 28 sont fermées, les vannes 26, 16 et 24 sont ouvertes, et ledit système de mise sous vide fournit une pression réduite
- dans l'autre phase, les vannes 16,26 et 24 sont fermées, les vannes 18, 25 et 28 sont ouvertes et ledit système de mise sous vide est arrêté.

De préférence, l'installation comporte en amont du ballon de flash un prétraitement comportant un moyen 4 de séparation des hydrocarbures, des condensats et des gaz et la canalisation 6 pour récupérer le solvant prétraité.

De préférence, l'installation comporte en outre une canalisation 5 reliée à la canalisation 6 pour l'introduction d'un agent chimique.

Avantageusement, l'installation comporte une canalisation 14 d'introduction de l'eau et de gaz dans un moyen 22 de séparation des gaz de ladite eau, ledit moyen comportant une canalisation 23 de sortie de l'eau et une canalisation 21 de sortie des gaz, ladite canalisation 21 étant reliée au système de mise sous vide 20 (compresseur par ex).

L'installation s'applique particulièrement bien lorsque le solvant est le monoéthylène glycol.

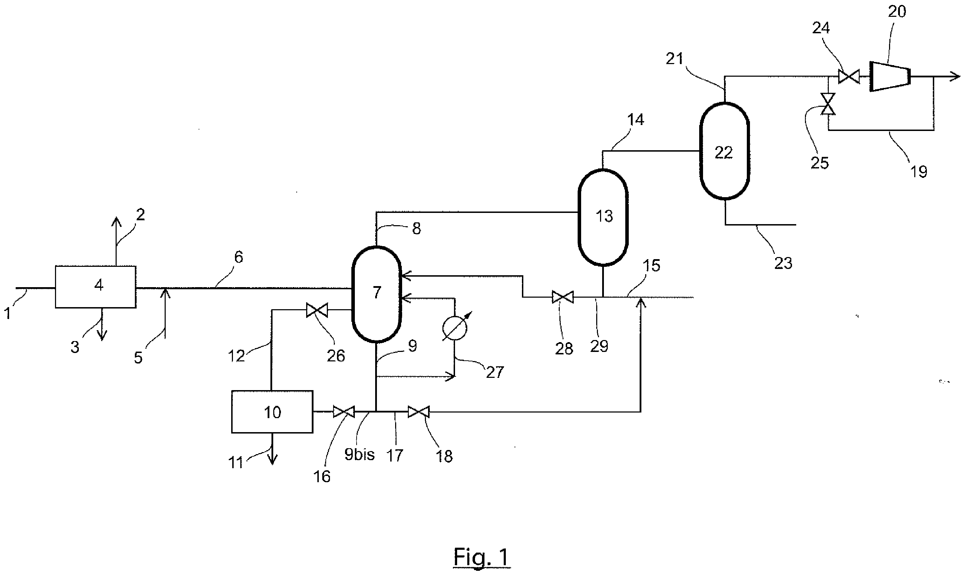

L'invention sera décrite à partir du schéma figure 1; le solvant est le MEG mais la figure peut être décrite avec tout autre solvant tel que défini dans l'invention ; on peut remplacer "MEG" par "solvant".

En amont du procédé proprement dit, il est avantageux d'opérer un prétraitement.

Le MEG à traiter (amené par la canalisation 1) est généralement séparé des gaz (sortant par la canalisation 2) et des condensats et hydrocarbures (sortant par la canalisation 3) dans un ballon 4. Il peut rester des traces ou de petites quantités d'hydrocarbures, de condensats ou de gaz selon le niveau de séparation.

Le MEG obtenu contient de l'eau et des sels. Il est optionnellement neutralisé, par ex par de la soude (amenée par la canalisation 5).

De préférence, le MEG est séparé des gaz, condensats et hydrocarbures, puis est neutralisé.

Le MEG est également préchauffé.

Le MEG à traiter contient une forte proportion d'eau (en général de 10 à 95%pds), une quantité de sels pouvant être élevée (de 0g/l à 90g/l par exemple, ou plus), le reste étant essentiellement du MEG.

On notera que le MEG à traiter ne subit pas de séparation des sels divalents. Les sels divalents restent présents dans le MEG entrant dans le ballon de flash décrit ci-après.

L'exploitant dispose d'un moyen de dosage de la quantité de sels dans le MEG à traiter.

Ce peut être un moyen manuel (sortie de l'échantillon au niveau d'un piquage et dosage) ou automatisé (mesure en ligne ou à partir d'un échantillon suivie d'un dosage et d'un asservissement).

Ce moyen est localisé au niveau du MEG à traiter, ou du MEG obtenu après séparation des gaz et des condensats, ou du MEG neutralisé, et de préférence au niveau du MEG neutralisé.

Lorsque la teneur en sels est supérieure au seuil de précipitation desdits sels dans le mélange entrant (à traiter ou prétraité), le procédé opère en phase de reclaiming, qui est décrite ci-après.

Le MEG à traiter contenant de l'eau et des sels, et de préférence issu du prétraitement , de préférence incluant la neutralisation, est envoyé par la canalisation 6 dans un ballon de séparation 7 .En fond de ballon, il sort un mélange de MEG et de sels. Il sort en tête du ballon (canalisation 8) un mélange de MEG et d'eau.

Cette séparation des sels est la première étape de la phase dite de reclaiming.

La séparation est opérée à une pression inférieure à la pression atmosphérique et préférentiellement comprise entre 0.2 et 0.5 bar absolus et à une température sensiblement égale au point d'ébullition du monéthylène glycol (ou plus généralement du solvant).

Pour atteindre la température voulue, des échangeurs thermiques peuvent être aménagés ainsi qu'un système de rebouilleur 27 sur le ballon (classique) . Ce système est très consommateur d'énergie puisque des quantités importantes de MEG et d'eau sont réchauffées et vaporisées.

En fond de ballon, on soutire le mélange MEG et sels (par la canalisation 9), puis les sels sont séparés à l'aide des moyens appropriés (moyen 10 pour séparer les sels). On pourra par exemple utiliser un ballon de sédimentation associé à une centrifugeuse, tout moyen connu de l'homme du métier convient. Les sels sont sortis du procédé (par la canalisation 11) et le MEG obtenu (généralement débarrassé des sels) est recyclé au flash (par la canalisation 12), après éventuel réchauffement.

Le MEG en mélange avec l'eau (de la canalisation 8) est distillé dans une colonne de distillation sous vide 13. L'eau est séparée en tête (sortant par la canalisation 14) et le MEG purifié est récupéré en fond de colonne (par la canalisation 15). Il peut alors être recyclé pour la production sur champ.

Cette distillation correspond à la deuxième étape du reclaiming.

Le vide est obtenu au moyen d'un système de mise sous vide 20 (tel qu'un compresseur), situé sur la canalisation 21 de soutirage des gaz issus de la distillation sous vide, lesdits gaz ayant été séparés de l'eau (sortant par la canalisation 23) par un moyen de séparation 22 situé sur la canalisation 14 d'évacuation de l'effluent de tête de la colonne de distillation 13 qui fonctionne sous vide.

Selon l'invention, lorsque la teneur en sels du MEG à traiter (ou prétraité) est inférieure au seuil de précipitation desdits sels , on opère la même installation de façon différente.

On arrête la phase de reclaiming et on met en oeuvre une phase dite de régénération.

Pour ce passage de la phase de reclaiming à la phase de régénération, il est prévu sur l'installation une vanne 16 sur la canalisation 9 et qui ferme l'accès au moyen de séparation 10 des sels, une canalisation 17 qui relie le fond du ballon 7 à la canalisation 15 de soutirage du MEG purifié, ladite canalisation 17 est munie d'une vanne 18. L'installation est également munie d'une canalisation 19 permettant de by-passer le système de mise sous vide 20.

Le procédé n'opère plus sous vide, le système de mise sous vide étant by-passé (vanne 24 fermée). La pression est établie à une pression égale ou supérieure à la pression atmosphérique et préférentiellement comprise entre 1 et 2 bar absolus.

Le MEG entrant dans le ballon de flash est séparé à une température inférieure à la température de dégradation du MEG (ou plus généralement du solvant) en un premier flux de monoéthylène glycol purifié et un flux de monoéthylène glycol et d'eau. Ledit premier flux sort en fond de ballon, et, la vanne 16 étant fermée, la vanne 18 étant ouverte, il est transféré par la canalisation 17 vers la canalisation 15.

Ledit flux de monoéthylène glycol et d'eau est distillé pour séparer l'eau et des gaz restants (sortant par la canalisation 14) et récupérer un deuxième flux de monoéthylène glycol purifié, sortant par la canalisation 15.

Dans un cas, lesdits premier et deuxième flux sont mélangés et évacués pour être réutilisés comme solvant. La proportion des flux dépendra des conditions opératoires, de la qualité de la séparation du flash et du niveau de purification souhaité.

Dans un autre cas, ledit deuxième flux est éventuellement recyclé vers l'étape de flash (ballon 7) par la canalisation 29. La vanne 28 autorise ou non ce recyclage. Cette vanne est fermée dans la phase de reclaiming.

De préférence, une partie dudit deuxième flux est mélangé audit premier flux. Le mélange est réutilisé comme solvant. L'autre partie dudit deuxième flux est recyclé à l'étape de flash (ballon 7).

Le niveau de purification du MEG souhaité et obtenu est supérieur à 60 % pds et préférentiellement supérieur à 80 % pds.

L'eau issue du procédé contient généralement moins de 1 % pds de MEG et préférentiellement moins de 0.1 % pds ; elle peut être réutilisée.

Il apparaît clairement que ce procédé permet des gains d'énergie importants (gain au niveau du système de rebouilleur 27 du ballon de séparation 7 et du système de mise sous vide 20 (tel qu'un compresseur) et il permet une grande flexibilité au niveau de l'exploitation, le passage d'une phase à l'autre restant très simple.

Un avantage est de pouvoir opérer en manuel ou en automatisé, avec un asservissement adapté des vannes selon les dosages de sels.

Ce procédé permet un excellent taux de récupération du solvant (en particulier MEG) qui est de plus de 99,5%, les pertes en MEG sont donc très minimisées.

A titre d'exemple, dans le cas où le MEG à traiter contient 70% pds de MEG et 30 % pds d'eau, le MEG purifié contenant 80 % pds de MEG et 20% pds d'eau. Le procédé en phase de régénération consomme 30% de l'énergie consommée par le procédé en phase de reclaiming ce qui procure un gain en énergie significatif.

Le procédé et l'installation décrits pour le MEG conviennent pour les autres solvants utilisés dans le traitement de gaz tels que des alcanolamines, et par exemple la monoéthanolamine (MEA), la diéthanolamine (DEA), ou la methyldiéthanolamine (MDEA).

## Revendications

1. Procédé de purification d'un solvant inhibiteur de la formation d'hydrates , ledit solvant ayant un point d'ébullition supérieur à celui de l'eau et étant, à un moment au moins, en mélange avec de l'eau et des sels, procédé qui comprend:
- éventuellement, un prétraitement dudit solvant à traiter, ledit prétraitement séparant au moins en partie les hydrocarbures, les condensats et les gaz, et ledit prétraitement comportant éventuellement d'addition d'agent chimique de neutralisation,
- une phase de reclaiming mise en oeuvre lorsque la teneur en sels dudit solvant à traiter, éventuellement prétraité, atteint le seuil de précipitation dans le mélange traité
- ladite phase comprenant un flash sous vide, opérant sous une pression inférieure à la pression atmosphérique et à une température inférieure à la température de dégradation du solvant, par lequel il est obtenu un flux de solvant contenant les sels et un flux vaporisé de solvant et d'eau, ledit flux de solvant et d'eau est distillé sous vide, à une pression sensiblement égale à celle régnant dans le dit flash sous vide pour séparer l'eau et récupérer un flux de solvant purifié, les sels sont séparés dudit flux de solvant contenant les sels, puis le solvant obtenu est recyclé au flash,
- lorsque ladite teneur en sels dans le mélange traité est inférieure au seuil de précipitation , la phase de reclaiming est arrêtée et une phase dite de régénération est mise en oeuvre,
- ladite phase de régénération comprenant la suppression du vide, le solvant à traiter, éventuellement prétraité, subissant une étape de flash , opérant sous une pression égale ou supérieure à la pression atmosphérique et à une température inférieure à la température de dégradation du solvant, par laquelle il est obtenu un premier flux de solvant purifié et un flux de solvant et d'eau, ledit flux de solvant et d'eau est distillé sous une pression sensiblement égale à celle régnant dans ledit flash , l'eau est séparée et un deuxième flux de solvant purifié est obtenu, ledit deuxième flux étant mélangé au dit premier flux, et/ou recyclé au dit flash.

2. Procédé selon la revendication 1 dans lequel le solvant est le monoéthylène glycol.

3. Procédé selon la revendication 1 dans lequel le solvant est une alcanolamine.

4. Procédé selon la revendication 3 dans lequel le solvant est choisi dans le groupe formé par la monoéthanolamine (MEA), la diéthanolamine (DEA), la methyldiéthanolamine (MDEA).

5. Procédé selon l'une des revendications précédentes dans lequel le solvant à traiter ne subit pas de traitement pour séparer les sels divalents.

6. Procédé selon l'une des revendications précédentes dans lequel, dans la phase de régénération, la pression du flash est comprise entre 1 et 2 bar absolus.

7. Procédé selon l'une des revendications précédentes dans lequel, dans la phase de reclaiming, la pression du flash sous vide est comprise entre 0.2 et 0.5 bar absolus.

8. Installation de purification d'un solvant inhibiteur de la formation d'hydrates , ledit solvant ayant un point d'ébullition supérieur à celui de l'eau et étant ,à un moment au moins, en mélange avec de l'eau et des sels, ladite installation comprenant:
- un ballon de flash 7 muni d'une canalisation 6 d'introduction du solvant à traiter, d'une canalisation 8 de sortie d'un mélange de solvant et d'eau, d'une canalisation 9 de sortie d'un mélange de solvant et de sels,
- un moyen 10 de séparation des sels muni d'une canalisation 9bis d'introduction du mélange de solvant et de sels, ladite canalisation 9bis comportant une vanne 16, ledit moyen 10 étant également muni d'une canalisation 11 de sortie des sels séparés et d'une canalisation 12 de sortie du solvant séparé des sels, cette dernière canalisation 12 étant reliée au ballon de flash 7 et munie d'une vanne 26,
- une colonne de distillation 13 pouvant opérer sous vide, munie d'une canalisation d'introduction 8 dudit mélange de solvant et d'eau, d'une canalisation de sortie 15 du solvant purifié située en fond de colonne, d'une canalisation de sortie 14 de l'eau et de gaz, située en tête de colonne,
- une canalisation 17 munie d'une vanne 18 reliant ladite canalisation 9 de sortie d'un mélange de solvant et de sels à la canalisation 15 de sortie du solvant purifié
- un système de mise sous vide 20 pouvant délivrer une pression réduite dans la colonne de distillation et le ballon de flash, une canalisation 19 de by-pass dudit système et des vannes 24 et 25 au niveau respectivement dudit système et de la canalisation de by-pass,
- une canalisation 29 munie d'une vanne 28, reliant la canalisation 15 de sortie du solvant purifié au ballon de flash 7,
et ladite installation fonctionnant selon 2 phases selon la teneur en sels du solvant délivrée par le moyen de dosage:
- dans l'une des phases, sont fermées la vanne de la canalisation reliant 18 la canalisation de sortie d'un mélange de solvant et de sels 9 à la canalisation de sortie du solvant purifié 15, et la vanne 25 située au niveau de la canalisation de by-pass et la vanne 28 située sur la canalisation qui relie la canalisation de sortie du solvant purifié 15 au ballon de flash alors que sont ouvertes la vanne 26 située sur la canalisation de sortie du solvant séparé des sels 12, la vanne 16 située sur la canalisation d'introduction du mélange de solvant et de sels 9bis et la vanne 24 située au niveau du système de mise sous vide , et ledit système de mise sous vide fournit une pression réduite
- dans l'autre phase, sont fermées la vanne 26 située sur la canalisation de sortie du solvant séparé des sels 12, la vanne 16 située sur la canalisation d'introduction du mélange de solvant et de sels 9bis et la vanne 24 située au niveau du système de mise sous vide, et sont ouvertes la vanne 18 de la canalisation reliant la canalisation de sortie d'un mélange de solvant et de sels 9 à la canalisation de sortie du solvant purifié 15, et la vanne 25 située au niveau de la canalisation de by-pass et la vanne 28 située sur la canalisation qui relie la canalisation de sortie du solvant purifié 15 au ballon de flash 7 et ledit système de mise sous vide est arrêté.

9. Installation selon la revendication 8, comportant en amont du ballon de flash, un moyen 4 de séparation des hydrocarbures, des condensats et des gaz.

10. Installation selon la revendication 8 ou 97, comportant une canalisation 5 pour l'introduction d'un agent chimique de neutralisation.

11. Installation selon l'une des revendications 8 à 10, comportant une canalisation 14 d'introduction de l'eau et de gaz dans un moyen 22 de séparation des gaz de ladite eau, ledit moyen comportant une canalisation 23 de sortie de l'eau et une canalisation 21 de sortie des gaz, cette dernière canalisation 21 étant reliée au système de mise sous vide 20.

## Patentansprüche

1. Verfahren zum Reinigen eines Lösungsmittels, das die Bildung von Hydraten hemmt, wobei das Lösungsmittel einen höheren Siedepunkt als Wasser aufweist und mindestens zu einem Zeitpunkt mit Wasser und Salzen gemischt ist, wobei das Verfahren Folgendes umfasst:
- gegebenenfalls eine Vorbehandlung des zu behandelnden Lösungsmittels, wobei die Vorbehandlung mindestens teilweise die Kohlenwasserstoffe, die Kondensate und die Gase trennt, und wobei die Vorbehandlung gegebenenfalls die Zugabe eines chemischen Neutralisationsmittels umfasst,
- eine Reclaiming-Phase, die durchgeführt wird, wenn der Salzgehalt des gegebenenfalls vorbehandelten zu behandelnden Lösungsmittels den Fällungsgrenzwert in dem behandelten Gemisch erreicht,
- wobei diese Phase ein Entspannungsvakuum umfasst, das unter einem Druck unterhalb des Atmosphärendrucks und bei einer Temperatur unterhalb der Zersetzungstemperatur des Lösungsmittels arbeitet, wodurch ein Lösungsmittelstrom, der die Salze enthält, und ein Dampfstrom aus Lösungsmittel und Wasser erhalten wird, wobei der Strom aus Lösungsmittel und Wasser unter Vakuum destilliert wird, bei einem Druck, der im Wesentlichen gleich dem im Entspannungsvakuum herrschenden ist, um das Wasser abzutrennen und einen gereinigten Lösungsmittelstrom zurückzugewinnen, wobei die Salze von dem Lösungsmittelstrom, der die Salze enthält, getrennt werden, wobei dann das erhaltene Lösungsmittel zur Entspannung recycelt wird,
- wenn der Salzgehalt in dem behandelten Gemisch unterhalb des Fällungsgrenzwerts liegt, die Reclaiming-Phase gestoppt wird und eine so genannte Regenerationsphase durchgeführt wird,
- wobei die Regenerationsphase die Aufhebung des Vakuums umfasst, wobei das gegebenenfalls vorbehandelte zu behandelnde Lösungsmittel einem Entspannungsschritt unterzogen wird, der unter einem Druck, der gleich oder größer als der Atmosphärendruck ist, und bei einer Temperatur unterhalb der Lösungsmittelzersetzungstemperatur arbeitet, durch den ein erster gereinigter Lösungsmittelstrom und ein Strom aus Lösungsmittel und Wasser erhalten wird, wobei der Strom aus Lösungsmittel und Wasser unter einem Druck destilliert wird, der im Wesentlichen gleich dem in der Entspannung herrschenden ist, das Wasser abgetrennt wird und ein zweiter gereinigter Lösungsmittelstrom erhalten wird, wobei der zweite Strom mit dem ersten Strom gemischt und/oder zur Entspannung recycelt wird.

2. Verfahren nach Anspruch 1, wobei das Lösungsmittel Monoethylenglycol ist.

3. Verfahren nach Anspruch 1, wobei das Lösungsmittel ein Alkanolamin ist.

4. Verfahren nach Anspruch 3, wobei das Lösungsmittel ausgewählt ist aus der Gruppe bestehend aus Monoethanolamin (MEA), Diethanolamin (DEA), Methyldiethanolamin (MDEA).

5. Verfahren nach einem der vorhergehenden Ansprüche, wobei das zu behandelnde Lösungsmittel keiner Behandlung zum Abtrennen der zweiwertigen Salze unterzogen wird.

6. Verfahren nach einem der vorhergehenden Ansprüche, wobei in der Regenerationsphase der Entspannungsdruck im Bereich zwischen 1 und 2 Bar absolut liegt.

7. Verfahren nach einem der vorhergehenden Ansprüche, wobei in der Reclaiming-Phase der Vakuumentspannungsdruck im Bereich zwischen 0,2 und 0,5 Bar absolut liegt.

8. Anlage zum Reinigen eines Lösungsmittels, das die Bildung von Hydraten hemmt, wobei das Lösungsmittel einen höheren Siedepunkt als Wasser aufweist und mindestens zu einem Zeitpunkt mit Wasser und Salzen gemischt ist, wobei die Anlage Folgendes umfasst:
- eine Entspannungstrommel 7, die mit einer Leitung 6 zum Einführen des zu behandelnden Lösungsmittels, mit einer Leitung 8 zum Ausleiten eines Gemischs aus Lösungsmittel und Wasser, mit einer Leitung 9 zum Ausleiten eines Gemischs aus Lösungsmittel und Salzen versehen ist,
- eine Einrichtung 10 zum Abtrennen der Salze, das mit einer Leitung 9a zum Einführen des Gemischs aus Lösungsmittel und Salzen versehen ist, wobei die Leitung 9a ein Ventil 16 umfasst, wobei die Einrichtung 10 auch mit einer Leitung 11 zum Ausleiten der abgetrennten Salze und mit einer Leitung 12 zum Ausleiten des von den Salzen abgetrennten Lösungsmittels versehen ist, wobei diese letztgenannte Leitung 12 mit der Entspannungstrommel 7 verbunden und mit einem Ventil 26 versehen ist,
- eine Destülationssäule 13, die unter Vakuum arbeiten kann, die mit einer Leitung zum Einführen 8 des Gemischs aus Lösungsmittel und Wasser, mit einer Leitung zum Ausleiten 15 des gereinigten Lösungsmittels, die sich am Boden der Säule befindet, mit einer Leitung zum Ausleiten 14 des Wassers und des Gases, die sich am Kopf der Säule befindet, versehen ist,
- eine Leitung 17, die mit einem Ventil 18 versehen ist, das die Leitung 9 zum Ausleiten eines Gemischs aus Lösungsmittel und Salzen mit der Leitung 15 zum Ausleiten des gereinigten Lösungsmittels verbindet,
- ein System zum Beaufschlagen mit Vakuum 20, das einen reduzierten Druck in der Destillationssäule und der Entspannungstrommel bereitstellen kann, eine Leitung 19 zur Umgehung dieses Systems und Ventile 24 und 25 jeweils an dem System und der Umgehungsleitung,
- eine Leitung 29, die mit einem Ventil 28 versehen ist, das die Leitung 15 zum Ausleiten des gereinigten Lösungsmittels mit der Entspannungstrommel 7 verbindet,
und wobei die Anlage in 2 Phasen funktioniert, je nach dem Salzgehalt des Lösungsmittels, das von der Dosierungseinrichtung bereitgestellt wird:
- in einer der Phasen ist das Ventil der Leitung 18, die die Leitung zum Ausleiten eines Gemischs aus Lösungsmittel und Salzen 9 mit der Leitung zum Ausleiten des gereinigten Lösungsmittels 15 verbindet, und
- das Ventil 25, das an der Umgehungsleitung liegt, und das Ventil 28, das auf der Leitung liegt, die die Leitung zum Ausleiten des gereinigten Lösungsmittels 15 in die Entspannungstrommel 7 verbindet, geschlossen, während das Ventil 26, das auf der Leitung zum Ausleiten des von den Salzen getrennten Lösungsmittels 12 liegt, das Ventil 16, das auf der Leitung zum Einführen des Gemischs aus Lösungsmittel und Salzen 9a liegt, und das Ventil 24, das am System zur Beaufschlagung mit Vakuum liegt, geöffnet ist, wobei das System zur Beaufschlagung mit Vakuum einen reduzierten Druck bereitstellt,
- in der anderen Phase, ist das Ventil 26, das auf der Leitung zur Ausleitung des von den Salzen abgetrennten Lösungsmittels 12 liegt, das Ventil 16, das auf der Leitung zum Einführen des Gemischs aus Lösungsmittel und Salzen 9a liegt, und das Ventil 24, das am System zur Beaufschlagung mit Vakuum liegt, geschlossen und das Ventil 18 der Leitung, die die Leitung zum Ausleiten eines Gemischs aus Lösungsmittel und Salzen 9 mit der Leitung zum Ausleiten des gereinigten Lösungsmittels 15 verbindet,
- und das Ventil 25, das an der Leitung zur Umgehung liegt und das Ventil 28, das auf der Leitung liegt, die die Leitung zum Ausleiten des gereinigten Lösungsmittels 15 mit der Entspannungstrommel 7 verbindet, ist geöffnet, und das System zur Beaufschlagung mit Vakuum wird angehalten.

9. Anlage nach Anspruch 8, die stromaufwärts der Entspannungstrommel eine Einrichtung 4 zum Trennen der Kohlenwasserstoffe, der Kondensate und der Gase umfasst.

10. Anlage nach Anspruch 8 oder 9, die eine Leitung 5 zum Einführen eines chemischen Neutralisationsmittels umfasst.

11. Anlage nach einem der Ansprüche 8 bis 10, die eine Leitung 14 zum Einführen von Wasser und von Gas in eine Einrichtung 22 zum Trennen der Gase von dem Wasser, umfasst wobei die Einrichtung eine Leitung 23 zum Ausleiten des Wassers und eine Leitung 21 zum Ausleiten der Gase umfasst, wobei letztgenannte Leitung 21 mit dem System zur Beaufschlagung mit Vakuum 20 verbunden ist.

## Claims

1. A method of purifying a hydrate formation inhibitor solvent, said solvent having a higher boiling point than water and being, at one time at least, in admixture with water and salts, the method comprising:
- optionally, pretreating said solvent to be treated, said pretreatment separating at least partly the hydrocarbons, the condensates and the gases, and said pretreatment optionally comprising adding a chemical neutralizing agent,
- a reclaiming step carried out when the salt content of said solvent to be treated, optionally pretreated, reaches the precipitation threshold in the treated mixture,
- said step comprising vacuum flashing, operating at a pressure below atmospheric pressure and at a temperature below the solvent degradation temperature, leading to a solvent stream containing the salts and a vaporized solvent and water stream, said solvent and water stream is vacuum distilled, at a pressure substantially equal to that prevailing in said vacuum flash step so as to separate the water and to recover a purified solvent stream, the salts are separated from said salt-containing solvent stream, then the solvent obtained is recycled to the flash step,
- when said salt content in the treated mixture is below the precipitation threshold, the reclaiming step is stopped and a step referred to as regeneration step is carried out,
- said regeneration step comprising vacuum removal, the solvent to be treated, optionally pretreated, undergoing a flash step, operating at a pressure equal to or greater than atmospheric pressure and at a temperature below the solvent degradation temperature, leading to a first purified solvent stream and a solvent and water stream, said solvent and water stream is distilled at a pressure substantially equal to that prevailing in said flash step, the water is separated and a second purified solvent stream is obtained, said second stream being mixed with said first stream and/or recycled to said flash step.

2. A method as claimed in claim 1, wherein the solvent is glycol monoethylene.

3. A method as claimed in claim 1, wherein the solvent is an alkanolamine.

4. A method as claimed in claim 3, wherein the solvent is selected from the group made up of monoethanolamine (MEA), diethanolamine (DEA) and methyl-diethanalamine (MDEA).

5. A method as claimed in any one of the previous claims, wherein the solvent to be treated undergoes no treatment for separating the divalent salts.

6. A method as claimed in any one of the previous claims wherein, in the regeneration step, the pressure of the flash step ranges between 1 and 2 bar absolute.

7. A method as claimed in any one of the previous claims wherein, in the reclaiming step, the pressure of the vacuum flash step ranges between 0.2 and 0.5 bar absolute.

8. A plant for purifying a hydrate formation inhibitor solvent, said solvent having a higher boiling point than water and being, at one time at least, in admixture with water and salts, said plant comprising:
- a flash drum 7 provided with a pipe 6 for delivery of the solvent to be treated, a pipe 8 for discharge of a mixture of solvent and water, and a pipe 9 for discharge of a mixture of solvent and salts,
- a salt separation means 10 provided with a pipe 9a for delivery of the mixture of solvent and salts, said pipe 9a comprising a valve 16, said means 10 being also provided with a pipe 11 for discharge of the separated salts and a pipe 12 for discharge of the solvent separated from the salts, this pipe 12 being connected to flash drum 7 and provided with a valve 26,
- a distillation column 13 that can be operated under vacuum, provided with a pipe 8 for delivery of said mixture of solvent and water, a pipe 15 for discharge of the purified solvent, arranged at the column bottom, and a pipe 14 for discharge of the water and the gas, arranged at the column top,
- a pipe 17 provided with a valve 18 connecting said pipe 9 for discharge of a mixture of solvent and salts to pipe 15 for discharge of the purified solvent,
- a vacuum system 20 that can deliver a reduced pressure in the distillation column and the flash drum, a pipe 19 for bypassing said system, and valves 24 and 25 at said system and at the bypass pipe respectively,
- a pipe 29 provided with a valve 28, connecting pipe 15 for discharge of the purified solvent to flash drum 7,
and said plant operating in 2 steps depending on the salt content of the solvent provided by the metering means:
- in one of the steps, valve 18 connecting pipe 9 for discharge of a mixture of solvent and salts to pipe 15 for discharge of the purified solvent, as well as valve 25 arranged at the bypass pipe and valve 28 arranged on the pipe connecting pipe 15 for discharge of the purified solvent to flash drum 7 are closed, whereas valve 26 arranged on pipe 12 for discharge of the solvent separated from the salts, valve 16 arranged on pipe 9a for delivery of the mixture of solvent and salts, and valve 24 arranged at the vacuum system are open, and said vacuum system supplies a reduced pressure,
- in the other step, valve 26 arranged on pipe 12 for discharge of the solvent separated from the salts, valve 16 arranged on pipe 9a for delivery of the mixture of solvent and salts, and valve 24 arranged at the vacuum system are closed, and valve 18 of pipe 9 connecting pipe 9 for discharge of a mixture of solvent and salts to pipe 15 for discharge of the purified solvent, valve 25 arranged at the bypass pipe and valve 28 arranged on the pipe connecting pipe 15 for discharge of the purified solvent to flash drum 7 are open, and said vacuum system is stopped.

9. A plant as claimed in claim 8 comprising, upstream from the flash drum, a means 4 for separating the hydrocarbons, the condensates and the gases.

10. A plant as claimed in any one of claims 8 or 9, comprising a pipe 5 for delivery of a chemical neutralizing agent.

11. A plant as claimed in any one of claims 8 to 10, comprising a pipe 14 for delivery of the water and the gases in a means 22 for separating the gases from said water, said means comprising a pipe 23 for discharge of the water and a pipe 21 for discharge of the gases, this pipe 21 being connected to vacuum system 20.
